Europäisches Patentamt

European Patent Office

Office européen des brevets

(11) Publication number: 0 213 728
A1

## (12) EUROPEAN PATENT APPLICATION

(21) Application number: 86305744.4

(51) Int. Cl.⁴: C12M 1/18 , B01L 3/14

(22) Date of filing: 25.07.86

(30) Priority: 29.07.85 GB 8519054

(43) Date of publication of application:
11.03.87 Bulletin 87/11

(84) Designated Contracting States:
AT BE CH DE FR GB IT LI NL SE

(71) Applicant: UNILEVER PLC
Unilever House Blackfriars P.O. Box 68
London EC4P 4BQ(GB)GB
Applicant: UNILEVER NV
Burgemeester s'Jacobplein 1 P.O. Box 760
NL-3000 DK
Rotterdam(NL)BECHDEFRITLINLSEAT

(72) Inventor: Jukes, Alan
25 Oaklands Avenue
West Wickham Kent BR4 9LF(GB)
Inventor: Swaine, Derwent
4 Northland Drive
Winchester Hants SO23 7AR(GB)

(74) Representative: Stancliffe, Terence
Christopher et al
UNILEVER PLC Patents Division P.O. Box 68
Unilever House
London EC4P 4BQ(GB)

(54) Methods and apparatus for chemical/biological testing.

(57) A test device for carrying out a test involving a liquid sample and an active material carried by a disc or strip of sheet material, comprising a body member having therein a liquid-receiving well, said well having a wall or bottom formed of transparent material to allow optical analysis of liquid contents of the well therethrough, and said body member also having therein at least one recessed formation constituting a slideway to receive and hold in slotwise manner a disc or strip of paper or other sheet carrying active material to participate in the test, wherein the slideway is in open communication with the liquid-receiving well, whereby said disc or strip when located in said slideway can contact the liquid sample received in said well, and allow optical analysis of said liquid sample through said transparent wall or bottom of said well.

Fig.3.

## Methods and Apparatus for Chemical/Biological Testing

Field of Invention

The present invention relates to methods and apparatus for chemical/biological testing, and to receptacles and devices for use in such testing, e.g. of active substances such as antibiotics. In particular examples, the invention provides receptacles for use in the testing of substances (for example microbial samples) with paper discs or strips impregnated with chemical reagents (for example antibiotics). The invention also provides devices and arrangements for loading paper discs into such receptacles. The invention is particularly, although not exclusively, concerned with receptacles, and devices for loading such receptacles, intended for use in antibiotic susceptibility testing of microorganisms.

Background of the Invention

One conventional method for testing the effect of an antibiotic on a microorganism involves adding to a petri dish containing a nutrient solid medium, seeded with the microorganism, a predetermined dose of an antibiotic, incubating the dish and then examining the resulting culture for inhibition of growth at and around the position of the antibiotic addition. In order to facilitate testing, and to enhance accuracy in adding small predetermined doses of antibiotics, the use of commercially prepared antibiotic susceptibility testing discs has become widespread. The prior art includes various devices for handling and delivering such discs, including for example USP 3 863 426 (Courvalin : Anvar), USP 3 934 753 (Curtiss : Pfizer).

In order to facilitate examination of the effects of an antibiotic on microorganism growth, the use of petri dishes, as the test receptacles, has been superseded largely by the use of receptacles containing small wells formed of transparent plastic materials. Into each of these wells is added a sample of microorganism and a measured amount of antibiotic. After a suitable incubation period, the amount of growth of the microorganism (if any) indicated by the turbidity of the liquid in the well, can be read by eye or by machine.

Examples of such receptacles and wells are shown in USP 4 239 853 and 4 076 592 (R L Bradley), which disclose apparatus for testing the action of antibiotics on bacterial suspensions, in which a common reservoir, for a bacterial suspension to be tested, can be made to deliver a portion of such suspension to each of a row of separate test chambers containing antibiotics, by tilting the device so that an appropriate portion of suspension is delivered to each test chamber by overflowing a respective barrier of limited height forming one side of the test chamber.

Unfortunately, in these procedures, if the antibiotic is added to the well by the use of an impregnated disc, the turbidity of the liquid cannot be read, by eye or by machine unless the disc is first removed from the well. The removal of discs from large numbers of wells in an automated system is inconvenient and may lead to contamination.

Summary of the Invention

The present invention aims to provide test methods and devices including receptacles which enable such tests to be carried out by more convenient procedures.

According to an aspect of the present invention, there is provided a receptacle for use in the testing of a substance with a disc or strip of paper or other sheet carrier material impregnated with or otherwise carrying a chemical reagent, which comprises a body, having a well formed of a transparent material, and a slot for receiving and retaining a paper disc, the slot having a depth substantially equal to the depth of the well and, wherein, at or near their bottoms, the well and the slot are in open communication.

According to an aspect of the present invention, a test device for carrying out a test involving a liquid sample and an active material carried by a disc or strip of sheet material, comprises a body member having therein a liquid-receiving well, said well having a wall or bottom formed of transparent material to allow optical analysis of liquid contents of the well therethrough, and said body member also having therein at least one recessed formation constituting a slideway to receive and hold in slotwise manner a disc or strip of paper or other sheet carrying active material to participate in the test, wherein the slideway is in open communication with the liquid-receiving well, whereby said disc or strip when located in said slideway can contact the liquid sample received in said well, and allow optical analysis of said liquid sample through said transparent wall or bottom of said well.

Typically, the well is slightly deeper than the slideway or slot. However, if this is the case, the well and the slideway or slot will communicate at a level such that, when the receptacle is in use and an amount of liquid is placed in the well, the level of communication between the well and slot will always be below the level of liquid in the well. Preferably, the well communicates with the slideway or slot for the whole depth of the slot. However, in such a case, the opening of the slot into the well will have a width substantially less than the diameter of a paper disc suitable to be used so that such a disc, when placed in the slot, cannot be displaced into the well. Preferably, the length of the slot is only slightly greater than the diameter of the discs it is to receive.

Preferably, the slot or slideway is orientated so that a paper disc or strip held slotwise therein is orientated transversely to said transparent wall or bottom of said well, so that test liquid in said well can be seen through wall or bottom with said disc or strip in non-obstructing edgewise view.

In a preferred embodiment, the receptacle body comprises two or more wells, each well having a slideway or slot, as described above, with which it communicates. In a particularly preferred embodiment, the receptacle body comprises a tray formed of a transparent plastics material which tray contains a plurality of side-by-side wells, each well being provided with a slot for receiving and retaining a paper disc. Such a tray can conveniently be provided with a reservoir area, for filling test liquid into the wells, which can be for example separated into zones, corresponding to the number of wells, by a series of barriers, as described in US 4 076 592.

The invention in another aspect also provides a loading device for loading a paper disc into a receptacle of the type described above, which device comprises an ejector for ejecting a disc from the end of a stack of such discs and through an exit aperture in the loading device along a path from the end of the stack to the exit aperture which is substantially at right angles to the stack. Typically, the means for ejecting the disc comprises a means for applying orthogonal pressure to the disc by means of a slideable prong or rod.

When the device is in use to load a paper disc into a receptacle of the type described above, the receptacle is held against the device such that the exit aperture in the device is in alignment with the disc receiving slot provided in the receptacle. For this reason, the device preferably has a casing which is provided with means for securing a receptacle during loading such that the disc receiving slot in the receptacle and the exit aperture are in alignment. Preferably, the receptacle is provided with lugs or flanges which engage with complementary formations on the loading device, for example by the provision on the device of a slideway and on the receptacle of flanges which are slideable in the slideway on the device, wherein the slideway or the flanges on the receptacle have stop means for halting movement of the receptacle when the disc receiving slot is aligned with the exit aperture of the device.

In a further aspect, the invention provides a device for loading paper discs into a receptacle of the type described above having a plurality of side-by-side wells, which device comprises a means for simultaneously ejecting a disc from the end of each of a plurality of stacks of discs and through each of a plurality of exit apertures in the device, each path from the end of a stack to the corresponding exit aperture being substantially at right angles to the stack.

The invention further provides a method of testing a liquid containing a (e.g. suspended) microorganism comprising:-

a) loading, into a disc-receiving slideway or slot provided in a test receptacle according to the invention and as described above, a disc or strip of paper or other sheet carrier material impregnated with or otherwise carrying a chemical reagent (e.g. comprising a microbial growth promotor or inhibitor such as an antibiotic);

b) adding to the receptacle an amount of liquid containing microorganism to be tested such that the level of liquid in the well associated with the disc receiving slot, lies above the (minimum) level of communication between the well and the slot; and

c) after incubation for a suitable period of time, analysing the liquid in the well, e.g. by eye or photometrically. Such a method can for example be a microbial culture test to determine sensitivity of said microbe to antibiotic, or to determine a microbiological growth characteristic thereof for attempting its identification.

The paper discs referred to are impregnated with a chemical reagent. A liquid substance containing the microorganism to be tested is placed in a well after a paper disc impregnated with a suitable reagent has been loaded into the slot in the receptacle. The liquid substance contacts the disc via the communication between the well and the slot and the reagent can diffuse into the liquid substance. A particularly important area of use of the receptacle and the loading device of the invention is in antibiotic susceptibility testing of microorganisms, such as bacteria, and in microbial identification testing. In such use, the paper discs are impregnated with one or more antibiotics or other microbial growth promoters or inhibitors. Uses of such antibiotic discs are described for example in J.Clin.Pathology, 1978, Vol. 31, pp 855-858.

## Detailed description of embodiments of the invention

In order to illustrate the invention further, embodiments thereof are described below by way of example only and with reference to the accompanying drawings, in which:-

Figure 1 is a plan view of a receptacle according to the invention;

Figure 2 is a front view of the receptacle of Figure 1;

Figure 3 is a side view of the receptacle of Figure 1;

Figure 4 is a plan view of a receptacle according to the invention having eight wells;

Figure 5 is a sectional view along the line A-A of Figure 4;

Figures 6 and 7 are side and plan views respectively of disc loading device according to the invention in which part of the casing is cut away to show internal details; and

Figure 8 is a side view of the device of Figure 6, which is provided with a cartridge containing a stack of impregnated paper discs, wherein the device is shown during use immediately after a disc has been loaded into a receptacle of Figure 1.

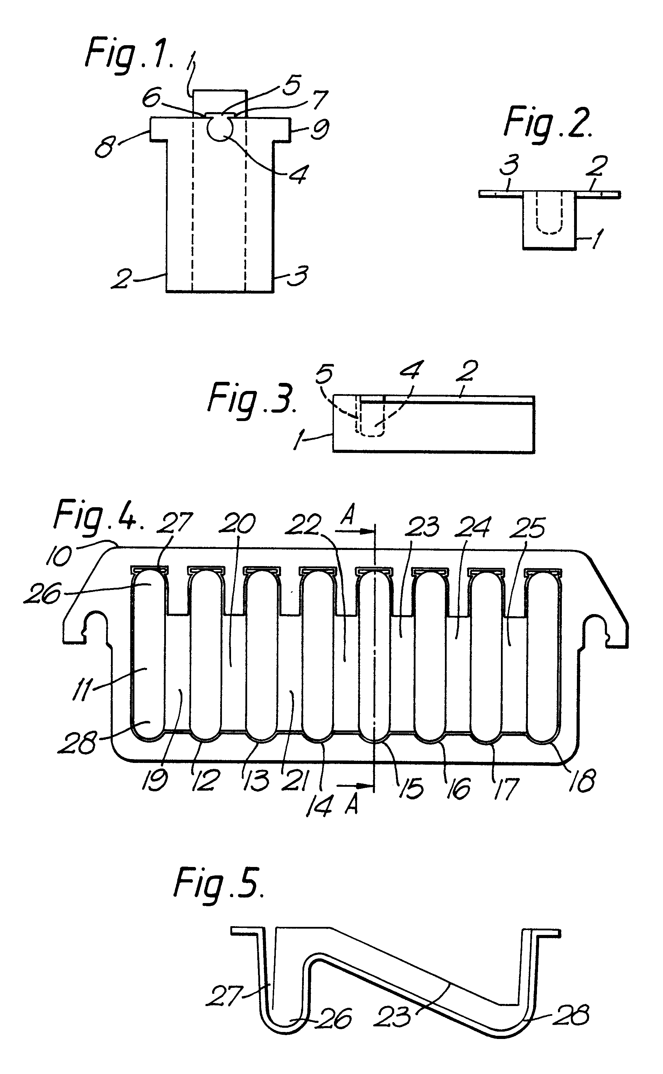

Referring to Figures 1 to 3, the receptacle comprises a body 1 having L-shaped flanges 2 and 3. The body contains a well 4 and a slot 5. The well has a depth slightly greater than the depth of the slot and the slot is in open communication for the whole of its depth with the well. Displacement of a disc (not shown) from the slot 5 into the well 4 is prevented by the flanges 6 and 7. The well 4 is formed of a transparent material, typically a plastics material so that, during use of the receptacle, the contents of the well can be examined by a photometric technique. Typically, for convenience, the body of the receptacle will be formed of a transparent plastics material. The flanges 2 and 3 on the sides of the body 1 are of a size such that they can slide in a suitable slideway provided on a disc loading device in preparation for the loading of a disc into slot 5. The flanges 2 and 3 have abutments 8 and 9, respectively, for holding the receptacle in such a slideway, provided on a loading device, at the correct loading position.

In the embodiment of Figures 4 and 5, the receptacle has a body 10 which is in the form of a tray divided into eight zones 11-18 by barriers 19-25. The tray is formed of a transparent plastics material. Each of the zones 11-18 comprises a well 26, a disc-receiving slot 27 and a reservoir 28. The reservoir 28 is provided to receive a substance to be tested, which substance is then transferred to the well for actual testing with a paper disc.

In the use of the device shown in Figures 4-5, a suspension of microorganisms containing for example about $10^5$ microorganisms per millilitre in a suitable medium (e.g. about 1-2 millilitre) can be introduced into reservoir 28, and aliquots of about 0.1 ml each may be transferred, by tilting the device, into the respective test chambers such as wells 26.

After incubation (e.g. for a few hours or overnight depending on the particular test in view) in the presence of the disc or strip of sheet carrier material (e.g. an impregnated 6 mm filter disc) the results are taken as mentioned below.

Measurement or observation of the test results obtained in the tests carried out by use of the devices as described herein can be done by eye or by photometry.

The devices as particularly set out and shown in Figures 1-5 of the drawings may be inspected or measured for their turbidity in the case of antibiotic sensitivity tests (breakpoint tests), or for various appropriate turbidity changes and/or colour changes in the case of culture tests to determine the bacteriological properties and identity of the microorganisms under test. Such inspection or measurement can be carried out by eye, by looking through the test liquid in each test well, along the axis of the substantially cylindrical well, with the filter discs (or strips) held in their slideways and seen substantially edgewise on, so as not to interfere with the view through the transparent bottom of the well.

In another embodiment of the invention, constituting a slight variant of the embodiment of Figures 4 and 5, an alternative slideway is provided in place of each disc-receiving slot 27. In the plan view of Figure 4, each slot 27 is seen to be positioned in such a way that a disc or strip, when held in said slot, is held in vertical orientation with its surface touching the periphery of the circular-section well 26. In the said slight variant (not shown in the drawing), the alternative slideway is provided in each substantially cylindrical well 26 in the form of two straight grooves, one extending up and down each of two (diametrically) opposite walls of the well, so that a paper disc or strip can be slid into the well and held there by its opposite edges in the grooves forming said slideway, in vertical orientation extending across a diameter of said well. The straight grooves can each be formed either as recesses in the wall of the well 26, or as a recess between a pair of spaced parallel ribs upstanding from the wall of well 26. In a preferred arrangement, such ribs are moulded along with the rest of the receptacle in transparent synthetic plastics material.

Also, in this variant it is preferred to accommodate paper discs in such slideways against a transparent and preferably hemispherical well bottom, so that in axial view along the well axis the disc is seen in edgewise view across a cross-sectional diameter, and lodged with one edge against the well bottom.

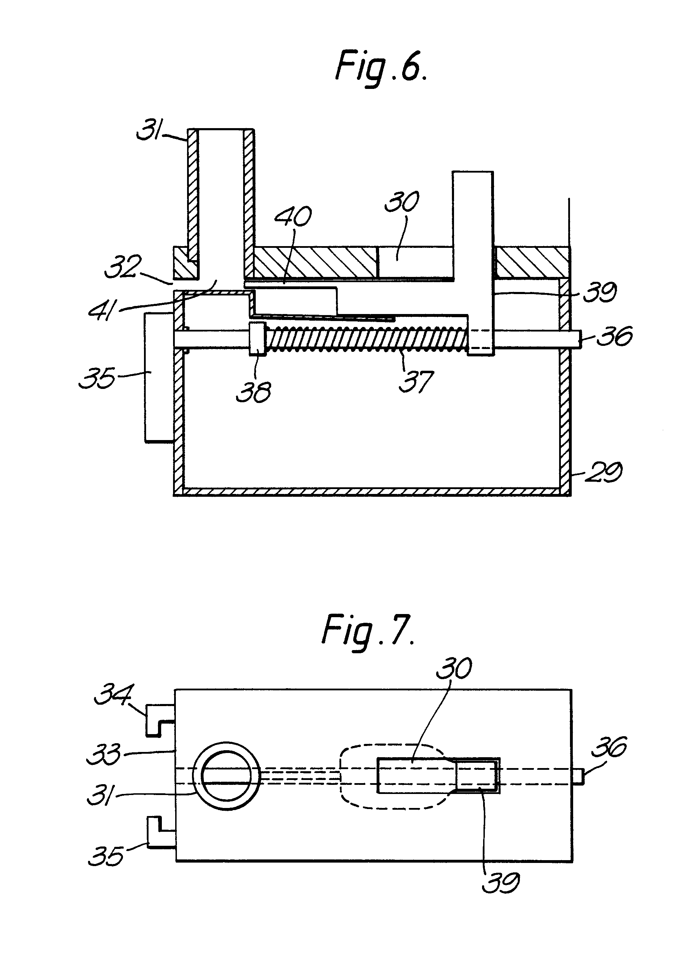

Referring to Figures 6 and 7, the device according to the invention comprises a substantially rectangular housing 29. The top of the housing has an opening 30 and a cylindrical cartridge holder 31, for holding a cartridge of paper. The front wall of the housing has an exit aperture 32 and a slideway 33 formed by guides 34 and 35. Inside the housing 29 there is a rod 36 fixed to the housing at each of its ends. A coil spring 37 is positioned on the rod 36. One end of the spring is attached to a fixed stop 38 and the other end of the spring is attached to a latch 39 which is slideably mounted on the rod 36. The latch 39 extends through the opening 30 of the housing. At the rest position of the spring, the latch rests against the back of the opening 30. The latch 39 extends laterally at its front into a prong 40. As the latch is pushed toward the front of the opening 30, the prong 40 is moved forward through a channel 41 toward the exit aperture 32. The movement of the latch forward is stopped when the latch meets the front of the opening 30. At this position the prong 40 projects through the aperture 32 and the spring is compressed. On release of the latch, the spring pushes the latch back to its rest position.

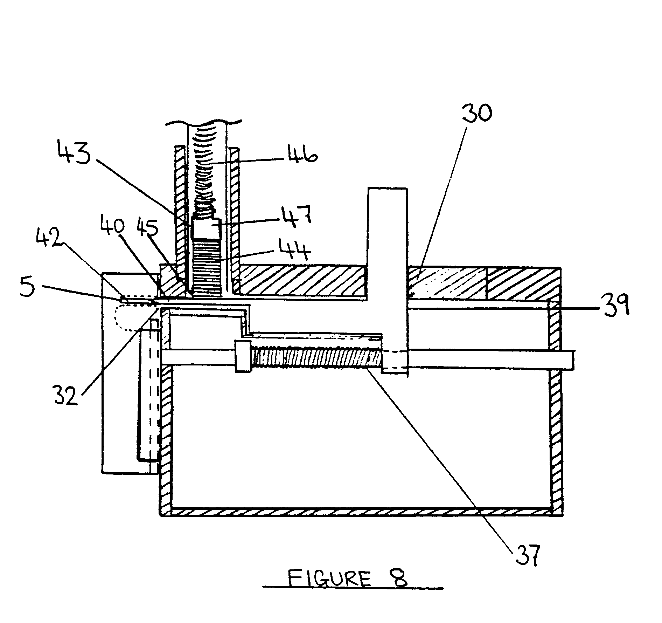

In Figure 8, the latch 39 abuts the front of the opening 30 and the spring 37 is compressed. The prong 40 projects through the aperture in the housing and is shown in the position where it has moved a disc 42 from the end of a cartridge 43 of discs, held in a cartridge holder 31, into the receiving slot 5 in the receptacle of Figure 1.

The discs to be dispensed are contained in the cylindrical cartridge 43. the discs are piled on top of one another in a stack 44 within the cartridge and are biassed towards one end 45 of the cartridge by means of a coil spring 46 and plunger 47. The other end of the cartridge (not shown) is closed by a cap. the end 45 of the cartridge which, in use, is the lower end, is open but is equipped with a pair of downwardly extended lugs (not shown) which extend slightly into the path of the discs to prevent movement of the bottom disc of the stack in a direction along the axis of the cartridge. However, the shape of the bottom edge on the end 45 of the cartridge is so positioned and shaped as to allow one disc at a time to be slid off the stack in a direction at right angles to the axis of the cartridge. The geometry is such that only movement in one direction is possible. The cartridge holder 31 and the cartridge 43 are provided with complementary formations such that insertion of the cartridge into the cartridge holder is possible only when the position of the cartridge is such that discs can leave the cartridge in a direction toward the aperture 32.

The present invention is susceptible of any modifications and variations as will be apparent to the skilled reader, and the present disclosure extends to the use of all combinations and subcombinations of the features described hereinabove and/or illustrated by the drawings.

### Claims

1. A test device for carrying out a test involving a liquid sample and an active material carried by a disc or strip of sheet material, comprising a body member having therein a liquid-receiving well, said well having a wall or bottom formed of transparent material to allow optical analysis of liquid contents of the well therethrough, and said body member also having therein at least one recessed formation constituting a slideway to receive and hold in slotwise manner a disc or strip of paper or other sheet carrying active material to participate in the test, wherein the slideway is in open communication with the liquid-receiving well, whereby said disc or strip when located in said slideway can contact the liquid sample received in said well, and allow optical analysis of said liquid sample through said transparent wall or bottom of said well.

2. A test device according to claim 1, wherein a receptacle body comprises two or more wells, each having a slideway or slot to receive and hold a said disc or strip.

3. A test device according to claim 2, wherein the body comprises a tray of transparent side-by-side wells each having said slideway or slot, and provided with a reservoir area for filling test liquid into said wells.

4. A test device according to claim 1, 2 or 3, wherein said slideway or slot is oriented to hold said disc or strip transversely to said transparent wall or bottom of said well, so that test liquid in said well can in use be seen through said wall or bottom with said disc or strip in edgewise view.

5. A test device according to claim 1, in combination with a loading device for loading a paper disc into said slot or slideway thereof, said loading device comprising an ejector for ejecting a disc from an end of a stack of such discs and through an exit aperture in said loading device along a path substantially at right-angles to said stack and into said slideway or slot.

6. A method of testing a liquid containing a microorganism, said method comprising the steps of a) loading into a disc-receiving slideway or slot provided in a test receptacle as defined in claim 1, a disc or strip of paper or other sheet carrier material carrying a chemical reagent active in said test; b) adding to the receptacle an amount of liquid containing microorganism to be tested, such that the level of liquid in the well associated with the slideway or slot is above the minimum level needed for communication between said well and said slot; and c) after incubation, optically analysing the liquid in said test receptacle.

7. Test devices and methods according to each preceding claim, characterised by any one or more of the features of the foregoing description and accompanying drawings.

Fig.1.

Fig.2.

Fig.3.

Fig.4.

Fig.5.

# Fig.6.

# Fig.7.

FIGURE 8

# EUROPEAN SEARCH REPORT

European Patent Office

Application number

EP 86 30 5744

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-B-2 343 987 (WALTER SARSTEDT KUNSTSTOFF-SPRITZGUSSWERK) * Claims; figures; column 2, line 62 - column 4, line 22 * | 1,4,7 | C 12 M 1/18<br>B 01 L 3/14 |
| A | FR-A-2 422 954 (N.B. CHITTY) * Figures; claims * | 1 | |
| D,A | US-A-4 239 853 (R.L. BRADLEY) * Figures; claims * | 1-3,7 | |
| D,A | FR-A-2 259 032 (PFIZER CO.) * Figures; claims * | 5,7 | |
| A | US-A-4 415 086 (J.T. BENNETT) * Figures * | 1-3,7 | |
| A | FR-A-2 468 643 (EIKEN CO. LTD.) * Figures; claims * | 1-3,7 | TECHNICAL FIELDS SEARCHED (Int. Cl.4)<br><br>C 12 M<br>B 01 L |

The present search report has been drawn up for all claims

| Place of search | Date of completion of the search | Examiner |
|---|---|---|
| THE HAGUE | 25-11-1986 | COUCKE A.O.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82